# EUROPEAN PATENT APPLICATION

(11) **EP 3 657 443 A1**
(43) Date of publication of application: **27.05.2020**
(21) Application number: 19210818.1
(22) Date of filing: 22.11.2019
(51) Int. Cl.: G06T 11/00

(54) **TOMOGRAPHIC IMAGE PROCESSING APPARATUS AND METHOD, AND COMPUTER PROGRAM PRODUCT**

(30) Priority: 22.11.2018 KR 20180145649
(71) Applicant: Samsung Electronics Co., Ltd., Gyeonggi-do 16677 (KR)
(72) Inventor: LEE, Kyoung-Young, 16677 Gyeonggi-do (KR); LEE, Donggue, 16677 Gyeonggi-do (KR); LEE, Duhgoon, 16677 Gyeonggi-do (KR)
(74) Representative: D'Halleweyn, Nele Veerle Trees Gertrudis

(57) **Abstract**

A tomographic image processing apparatus includes a data acquisition interface configured to acquire raw data; a memory; and at least one processor configured to: obtain, from the memory, a first partial reconstruction image corresponding to a partial angular range of a first rotation period of an X-ray generator; generate a second partial reconstruction image from partial raw data acquired in a partial angular range of a second rotation period of the X-ray generator, wherein the partial angular range of the first rotation period corresponds to the partial angular range of the second rotation period; generate a third partial reconstruction image based on the first partial reconstruction image and the second partial reconstruction image; store the third partial reconstruction image in the memory; and generate a resultant image based on the third partial reconstruction image and a plurality of partial reconstruction images stored in the memory.

## Description

### BACKGROUND

### 1. Field

The disclosure relates to a tomographic image processing apparatus, a tomographic image processing method, and a computer program product including instructions for performing the tomographic image processing method.

### 2. Description of Related Art

Medical imaging apparatuses may be used to obtain images showing an internal structure of an object. The medical imaging apparatuses may be non-invasive examination apparatuses that capture and process images of details of structures, tissue, fluid flow, etc., inside a body and display the images to a user. A user, for example a medical practitioner, may use medical images output from the medical imaging apparatuses to diagnose a patient's condition and diseases.

A computed tomography (CT) apparatus is an example of an apparatus for imaging an object by irradiating a patient with X-rays. A CT apparatus is a type of medical imaging apparatus or tomographic imaging apparatus. CT apparatuses are capable of providing a cross-sectional image of an object and may represent an internal structure, for example, organs such as a kidney, a lung, etc., of the object without superimposition of adjacent structures, as compared to a general X-ray apparatus. Due to these advantages, CT apparatuses are widely used for precise diagnosis of diseases.

### SUMMARY

Provided is a method and apparatus for providing an image obtained by reducing noise in a tomographic image generated using a partial image reconstruction technique.

Additional aspects will be set forth in part in the description which follows and, in part, will be apparent from the description, or may be learned by practice of the presented embodiments.

In accordance with an aspect of the disclosure, a tomographic image processing apparatus includes a data acquisition interface configured to acquire raw data; a memory; and at least one processor configured to: obtain, from the memory, a first partial reconstruction image corresponding to a partial angular range of a first rotation period of an X-ray generator; generate a second partial reconstruction image from partial raw data acquired in a partial angular range of a second rotation period of the X-ray generator, wherein the partial angular range of the first rotation period corresponds to the partial angular range of the second rotation period; generate a third partial reconstruction image based on the first partial reconstruction image and the second partial reconstruction image; store the third partial reconstruction image in the memory; and generate a resultant image based on the third partial reconstruction image and a plurality of partial reconstruction images stored in the memory.

The plurality of partial reconstruction images may respectively correspond to a plurality of angular ranges with a same angular interval therebetween, a sum of the plurality of angular ranges corresponding to the plurality of partial reconstruction images may correspond to an angular range of the resultant image, and the at least one processor may be further configured to generate the resultant image by summing the plurality of partial reconstruction images.

The memory may include a queue memory operating in a first-in-first-out (FIFO) mode, and a storage space corresponding to a capacity for storing a predetermined number of the plurality of partial reconstruction images, and the predetermined number may be a number of the partial reconstruction images used to generate the resultant image.

The memory may be configured to delete the first partial reconstruction image based on the third partial reconstruction image being input.

The at least one processor may be further configured to: register the first partial reconstruction image to the second partial reconstruction image; and generate the third partial reconstruction image by using the second partial reconstruction image and the first partial reconstruction image after the registering .

The at least one processor may be further configured to generate the third partial reconstruction image by performing averaging synthesis on the first partial reconstruction image and the second partial reconstruction image.

The resultant image may include a metal object.

The at least one processor may be further configured to: extract a first non-metal region from the first partial reconstruction image and a second non-metal region from the second partial reconstruction image; synthesize the first non-metal region with the second non-metal region; and generate the third partial reconstruction image by using an image obtained by the synthesizing and the second partial reconstruction image.

The at least one processor may be further configured to: detect motion information in the first partial reconstruction image and the second partial reconstruction image; and based on a motion value being greater than or equal to a predetermined reference value, store the second partial reconstruction image in the memory.

The at least one processor may be further configured to: acquire motion information indicating motion between the first partial reconstruction image and the second partial reconstruction image; and based on a motion value of the motion being greater than or equal to a preset reference value, generate the third partial reconstruction image by weighted averaging the first partial reconstruction image and the second partial reconstruction image, wherein a weight of the first partial reconstruction image is lower than a weight of the second partial reconstruction image.

The raw data may include image data regarding a moving body part, and the at least one processor may be further configured to: compensate for motion of the moving body part in the first partial reconstruction image, based on motion information of the moving body part; and generate the third partial reconstruction image based on the compensated first partial reconstruction image and the second partial reconstruction image.

The at least one processor may be further configured to perform a computed tomography (CT) fluoroscopy scan.

The partial angular range of the first rotation period may be less than an angular range of the resultant image.

In accordance with an aspect of the disclosure, a tomographic image processing method includes acquiring raw data; obtaining, from a memory, a first partial reconstruction image corresponding to a partial angular range of a first rotation period of an X-ray generator generating a second partial reconstruction image from partial raw data acquired in a partial angular range of a second rotation period of the X-ray generator, wherein the partial angular range of the first rotation period corresponds to the partial angular range of the second rotation period; generating a third partial reconstruction image based on the first partial reconstruction image and the second partial reconstruction image; storing the third partial reconstruction image in the memory; and generating a resultant image based on the third partial reconstruction image and a plurality of partial reconstruction images stored in the memory.

The plurality of partial reconstruction images may respectively correspond to a plurality of angular ranges with a same angular interval therebetween, a sum of the plurality of angular ranges corresponding to the plurality of partial reconstruction images may correspond to an angular range of the resultant image, and the generating of the resultant image may include generating the resultant image by summing the plurality of partial reconstruction images.

The memory may include a queue memory operating in a first-in-first-out (FIFO) mode and includes a storage space corresponding to a capacity for storing a predetermined number of the plurality of partial reconstruction images, and the predetermined number may be a number of the partial reconstruction images used to generate the resultant image.

The memory may be configured to delete the first partial reconstruction image based on the third partial reconstruction image being input.

In accordance with an aspect of the disclosure, a computer program comprising program instructions, wherein the program instructions, when executed by a processor, cause the processor to perform a tomographic image processing method comprising: acquiring raw data; obtaining, from a memory, a first partial reconstruction image corresponding to a partial angular range of a first rotation period of an X-ray generator; generating a second partial reconstruction image from partial raw data acquired in a partial angular range of a second rotation period of the X-ray generator, wherein the partial angular range of the first rotation period corresponds to the partial angular range of the second rotation period; generating a third partial reconstruction image based on the first partial reconstruction image and the second partial reconstruction image; storing the third partial reconstruction image in the memory; and generating a resultant image based on the third partial reconstruction image and a plurality of partial reconstruction images stored in the memory.

In accordance with an aspect of the disclosure, a tomographic image processing apparatus for performing a computed tomography (CT) fluoroscopy scan includes a data acquirer configured to acquire raw data; a memory; and at least one processor configured to: generate an intermediate resultant image from partial raw data acquired in an interval of one rotation period of an X-ray generator; and generate a new resultant image by synthesizing the intermediate resultant image and a previous resultant image stored in the memory and corresponding to at least one previous rotation period of the X-ray generator.

The at least one processor may be further configured to: obtain, from the memory, a first partial reconstruction image corresponding to a partial angular range of the at least one previous rotation period of the X-ray generator generate a second partial reconstruction image from the partial raw data acquired in a partial angular range of the one rotation period of the X-ray generator, wherein the partial angular range of the at least one previous rotation period corresponds to the partial angular range of the one rotation period; generate a third partial reconstruction image by using the first partial reconstruction image and the second partial reconstruction image; store the third partial reconstruction image in the memory; and generate the intermediate resultant image based on the third partial reconstruction image and a plurality of partial reconstruction images stored in the memory.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 illustrates a structure of a computed tomography (CT) system according an embodiment;
FIG. 2 illustrates a process of scanning an object by using CT fluoroscopy, according to an embodiment;
FIG. 3 is a block diagram of a structure of a tomographic image processing apparatus according to an embodiment;
FIG. 4 is a flowchart of a tomographic image processing method according to an embodiment;
FIG. 5 illustrates a process of acquiring raw data according to an embodiment;
FIG. 6 illustrates a process of generating a resultant image based on first and second partial reconstruction images, according to an embodiment;
FIG. 7 illustrates a process of performing registration and synthesis, according to an embodiment;
FIG. 8 illustrates a process of synthesizing a new partial angle reconstructed (PAR) image and an existing PAR image, according to an embodiment;
FIG. 9 is a flowchart of a process of registering and synthesizing partial reconstruction images, according to an embodiment;
FIG. 10 is a flowchart of a method of registering and synthesizing partial reconstruction images, according to an embodiment;
FIG. 11 illustrates a user interface (UI) view according to an embodiment;
FIG. 12 illustrates a UI view according to an embodiment;
FIG. 13 illustrates a process of synthesizing an intermediate resultant image and an existing resultant image, according to an embodiment; and
FIG. 14 illustrates an effect of a method according to an embodiment compared to a method of the related art when using simulation data acquired without motion.

### DETAILED DESCRIPTION

Principles of the disclosure are explained and embodiments are disclosed so that the present scope is clarified and one of ordinary skill in the art to which the disclosure pertains. The disclosed embodiments may have various forms.

Throughout the disclosure, the expression "at least one of a, b or c" indicates only a, only b, only c, both a and b, both a and c, both b and c, all of a, b, and c, or variations thereof.

Throughout the specification, like reference numerals or characters refer to like elements. In the present specification, all elements of embodiments are not explained, but general matters in the technical field of the present disclosure or redundant matters between embodiments will not be described. Terms 'module' or 'unit' used herein may be implemented using at least one or a combination from among software, hardware, or firmware, and, according to embodiments, a plurality of 'module' or 'unit' may be implemented using a single element, or a single 'module' or 'unit' may be implemented using a plurality of units or elements. An operational principle of the present disclosure and embodiments thereof will now be described more fully with reference to the accompanying drawings.

In the present specification, an image may include a medical image obtained by a medical imaging apparatus, such as a computed tomography (CT) apparatus, a magnetic resonance imaging (MRI) apparatus, an ultrasound imaging apparatus, or an X-ray apparatus.

Throughout the specification, the term 'object' may refer to a thing to be imaged, and may include a human, an animal, or a part of a human or animal. For example, the object may include a part of a body, for example an organ, a phantom, or the like.

In the present specification, a 'CT system' or 'CT apparatus' may refer to a system or apparatus configured to emit X-rays while rotating around at least one axis relative to an object and photograph the object by detecting the X-rays.

In the specification, a 'CT image' may refer to an image constructed from raw data obtained by photographing an object by detecting X-rays that are emitted as the CT system or apparatus rotates about at least one axis with respect to the object.

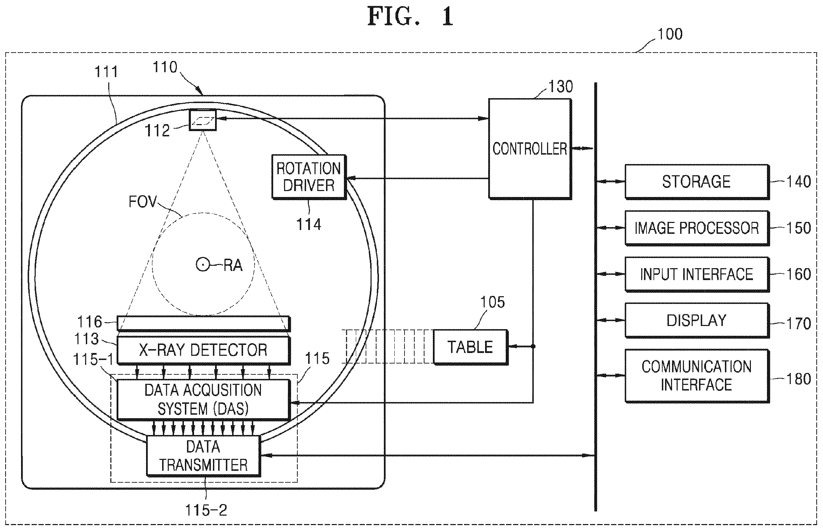

FIG. 1 illustrates a structure of a CT system 100 according to an embodiment.

The CT system 100 may include a gantry 110, a table 105, a controller 130, a storage 140, an image processor 150, an input interface 160, a display 170, and a communication interface 180.

The gantry 110 may include a rotating frame 111, an X-ray generator 112, an X-ray detector 113, a rotation driver 114, and a readout device 115.

The rotating frame 111 may receive a driving signal from the rotation driver 114 and rotate around a rotation axis (RA).

An anti-scatter grid 116 may be disposed between an object and the X-ray detector 113 and may transmit most primary radiation and attenuate scattered radiation. The object may be positioned on the table 105 which may move, tilt, or rotate during a CT scan.

The X-ray generator 112 receives a voltage and a current from a high voltage generator (HVG) to generate and emit X-rays.

The CT system 100 may be implemented as a single-source CT system including one X-ray generator 112 and one X-ray detector 113, or as a dual-source CT system including two X-ray generators 112 and two X-ray detectors 113.

The X-ray detector 113 detects radiation that has passed through the object. For example, the X-ray detector 113 may detect radiation by using a scintillator, a photon counting detector, etc.

Methods of driving the X-ray generator 112 and the X-ray detector 113 may vary depending on scan modes used for scanning of the object. The scan modes are classified into an axial scan mode and a helical scan mode, according to a path along which the X-ray detector 113 moves. Furthermore, the scan modes are classified into a prospective mode and a retrospective mode, according to a time interval during which X-rays are emitted.

The controller 130 may control an operation of each of the components of the CT system 100. The controller 130 may include a memory configured to store program for performing a function or data and a processor configured to process the program codes or the data. The controller 130 may be implemented in various combinations of at least one memory and at least one processor. The processor may generate or delete a program module according to an operating status of the CT system 100 and process operations of the program module.

The readout device 115 receives a detection signal generated by the X-ray detector 113 and outputs the detection signal to the image processor 150. The readout device 115 may include a data acquisition system (DAS) 115-1 and a data transmitter 115-2. The DAS 115-1 uses at least one amplifying circuit to amplify a signal output from the X-ray detector 113, and outputs the amplified signal. The data transmitter 115-2 uses a circuit such as a multiplexer (MUX) to output the signal amplified in the DAS 115-1 to the image processor 150. According to a slice thickness or a number of slices, only some of a plurality of pieces of data collected by the X-ray detector 113 may be provided to the image processor 150, or the image processor 150 may select only some of the plurality of pieces of data.

The image processor 150 obtains tomography data from a signal obtained by the readout device 115, for example, pure data that is data before being processed. The image processor 150 may pre-process the obtained signal, convert the obtained signal into tomography data, and post-process the tomography data. The image processor 150 may perform some or all of the processes described herein, and the type or order of processes performed by the image processor 150 may vary according to embodiments.

The image processor 150 may perform pre-processing, such as a process of correcting sensitivity irregularity between channels, a process of correcting a rapid decrease of signal strength, or a process of correcting signal loss due to an X-ray absorbing material, on the signal obtained by the readout device 115.

According to embodiments, the image processor 150 may perform some or all of the processes for reconstructing a tomographic image, to thereby generate the tomography data. According to an embodiment, the tomography data may be in the form of data that has undergone back-projection, or in the form of a tomographic image. According to embodiments, additional processing may be performed on the tomography data by an external device such as a server, a medical apparatus, or a portable device.

Raw data is a set of data values corresponding to intensities of X-rays that have passed through the object, and may include projection data or a sinogram. The data that has undergone back-projection is obtained by performing back-projection on the raw data by using information about an angle at which X-rays are emitted. The tomographic image is obtained by using image reconstruction techniques including back-projection of the raw data.

The storage 140 is a storage medium for storing control-related data, image data, etc., and may include a volatile or non-volatile storage medium.

The input interface 160 receives control signals, data, etc., from a user. The display 170 may display information indicating an operational status of the CT system 100, medical information, medical image data, etc.

The CT system 100 includes the communication interface 180 and may be connected to external devices, such as a server, a medical apparatus, and a portable device (smartphone, tablet personal computer (PC), wearable device, etc.), via the communication interface 180.

The communication interface 180 may include one or more components that enable communication with an external device. For example, the communication interface 180 may include a short distance communication module, a wired communication module, and a wireless communication module.

According to embodiments, the CT system 100 may or may not use contrast media during a CT scan, and may be implemented as a device connected to other equipment.

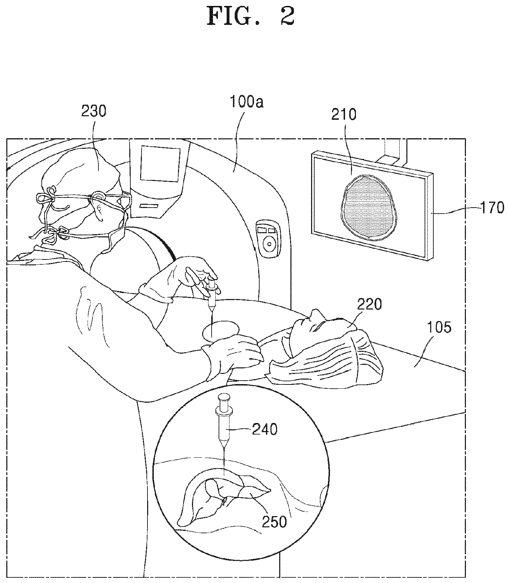

FIG. 2 illustrates a process of scanning an object by using CT fluoroscopy, according to an embodiment.

According to an embodiment, a CT system 100a, which may correspond to CT system 100 described above, reconstructs and provides a real-time CT image 210 while a user 230 performs surgery or a medical procedure on an object 220. The user 230 may receive the real-time CT image 210 by performing a CT scan at his or her desired time point. The real-time CT image 210 may be provided via a display 170. During the medical procedure, the user 230 may control a CT scan process and movement of a table 105 by using various input devices in the input interface, for example input interface 160 of FIG. 1. To achieve this, the input interface 160 may include a pedal, a button, a jog, a dial, a key, a touch screen, a touch pad, a wheel, etc. For example, the input interface 160 may include first and second pedals, and the user 230 may control the CT system 100a to perform a CT scan by pressing the first pedal and move the table 105 by pressing the second pedal. The CT system 100a may perform a CT scan while the first pedal is pressed down and may not perform the CT scan while the first pedal is not pressed down. Information about the progress of the CT scan may be provided via the display 170.

According to the embodiment, the CT system 100a may be used to perform CT fluoroscopy. The CT fluoroscopy may be used to monitor insertion of a surgical instrument 240, which may be needed for a guide biopsy procedure, cervical nerve root blocks, etc. For example, the user 230 may use the real-time CT image 210 as a guide to insert the surgical instrument 240 into a liver 250 in order to extract tissue from the liver 250. In this case, because the user 230 may check in real-time a position of the surgical instrument 240 inside a patient, so it may be important to provide the user 230 with near real-time image feedback. To accomplish this, according to embodiments, CT fluoroscopy may use dynamic image reconstruction algorithms other than existing CT reconstruction techniques. Dynamic image reconstruction is a method whereby images may be consecutively reconstructed from raw data acquired over an angular range during continuous scanning. In this case, partial reconstruction images reconstructed from raw data acquired in the angular range are used for image reconstruction. A partial reconstruction image has only information about the particular angular range, and thus provides only information about an object in a certain direction. In a CT fluoroscopic procedure, wherein a scan is performed while the user 230 inserts the surgical instrument 240 into a patient's body, for example for about 5 to about 660 seconds, both the user 230 and the patient may receive an excessive cumulative radiation dose, compared to existing CT scans. Thus, the CT fluoroscopy may be performed at a dose that is about one-sixth to about one-third of that used in a general CT scan. This results in a low image quality.

According to embodiments, a CT image is generated using partial reconstruction images, and a signal-to-noise (SNR) ratio of a new partial reconstruction image is improved by using a partial reconstruction image obtained over a previous period of a CT scan. Accordingly, an image quality and a SNR of a CT image may be improved.

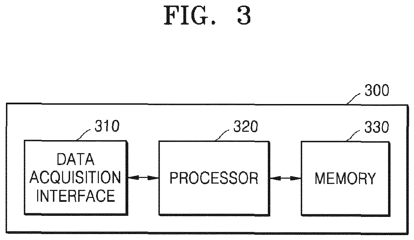

FIG. 3 is a block diagram of a structure of a tomographic image processing apparatus 300 according to an embodiment.

According to an embodiment, the tomographic image processing apparatus 300 may include a data acquisition interface 310, a processor 320, and a memory 330.

The tomographic image processing apparatus 300 may be implemented in the form of a CT system, a general-purpose computer, a portable terminal, or a kiosk. For example, the portable terminal may be implemented as a smartphone, a tablet personal computer (PC), etc. For example, the CT system may be implemented as the CT system 100 of FIG. 1 or the CT system 100a of FIG. 2.

The data acquisition interface 310 acquires raw data by scanning an object. The raw data may correspond to projection data or a sinogram. According to an embodiment, in the CT system, raw data is acquired by scanning an object in a prospective mode.

According to an embodiment, the data acquisition interface 310 may correspond to a scanner for acquiring raw data by scanning an object via X-rays. For example, the scanner may include the X-ray generator 112 and the X-ray detector 113 described with reference to FIG. 1. According to the embodiment, the data acquisition interface 310 may acquire raw data by scanning an object according to a protocol set under control by the processor 320.

According to another embodiment, the data acquisition interface 310 may correspond to a communication interface or an input/output (I/O) device via which raw data is acquired from an external device. Examples of an external device include a CT system, a medical data server, another user's terminal, etc. According to the embodiment, the data acquisition interface 310 may be connected to an external device via various wired or wireless networks such as a wired cable, a local area network (LAN), a mobile communication network, the Internet, etc. The data acquisition interface 310 may correspond to the communication interface 180 described with reference to FIG. 1.

The processor 320 may control all operations of the tomographic image processing apparatus 300 and process data. The processor 320 may include at least one processor. According to an embodiment, the processor 320 performs all operations of controlling a gantry, for example gantry 110 of FIG. 1, and processing raw data and may be implemented as one or a plurality of processors. According to another embodiment, the processor 320 may correspond to one or more processors for processing raw data received from an external device. The processor 320 may correspond to the image processor 150 of FIG. 1 or a combination of the image processor 150 and the controller 130.

The processor 320 may generate a plurality of partial reconstruction images, for example partial angle reconstructed (PAR) images, and a resultant image from raw data. A plurality of PAR images are reconstructed from raw data acquired at first angular intervals and represent information about an object in a part of the entire angular range of 360°. The first angular interval may be an angular range less than 360° For example, the resultant image may correspond to an angular range of 360°, and the first angular interval may be 60°. As another example, the resultant image may correspond to an angular range of 180°, and the first angular interval may be 60°.

When raw data is received from the data acquisition interface 310, the processor 320 generates PAR images at the first angular intervals and controls the memory 330 to store the generated PAR images.

The memory 330 may store a plurality of PAR images. According to an embodiment, the memory 330 is a queue memory operating in a first-in- first-out (FIFO) mode. The memory 330 is configured to store only a predetermined amount of image data. For example, the memory 330 may be configured to store six PAR images. Thus, when a new PAR image is input to the memory 330, the oldest PAR image may be deleted from the memory 330. The capacity of the memory 330 may be determined depending on an angular interval corresponding to a resultant image and the first angular interval. For example, when the resultant image corresponds to a 360 degree angular range and the first angular interval is 60 degrees, the memory 330 may have a storage space with a capacity for storing six PAR images.

The memory 330 may be formed as a volatile or non-volatile memory. According to an embodiment, the memory 330 may correspond to the storage 140 of FIG. 1.

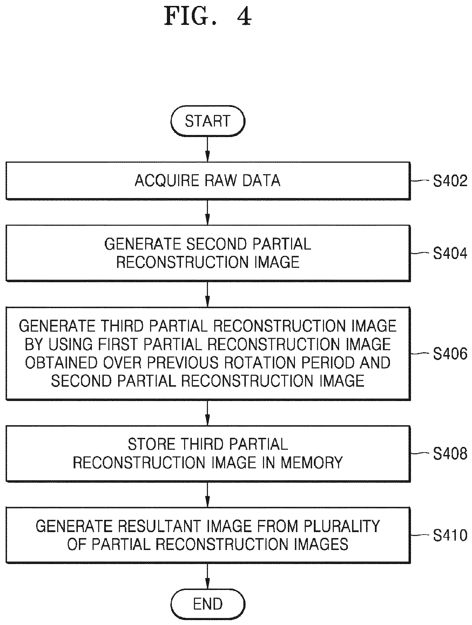

FIG. 4 is a flowchart of a tomographic image processing method according to an embodiment.

According to embodiments, operations of the tomographic image processing method may be performed by various electronic devices including at least one processor. The present disclosure includes an embodiment in which the tomographic image processing apparatus 300 according to the disclosure performs a method of controlling a tomographic image processing apparatus, according to the disclosure. Thus, embodiments described with respect to the tomographic image processing apparatus 300 may be applied to a tomographic image processing method, and embodiments described with respect to a tomographic image processing method may be applied to embodiments described with respect to the tomographic image processing apparatus 300. Although it has been described that tomographic image processing methods according to embodiments are performed by the tomographic image processing apparatus 300 according to the disclosure, embodiments are not limited thereto, and the tomographic image processing methods may be performed by various types of electronic devices.

First, the tomographic image processing apparatus 300 acquires raw data by scanning an object at operation S402. The raw data may be a sinogram or projection data.

Next, the tomographic image processing apparatus 300 generates a second partial reconstruction image from first raw data acquired over a first angular range at operation S404. The first angular range is an angular range having a preset first angular interval. The preset first angular interval may have an angular range less than that of a resultant image. The second partial reconstruction image may be reconstructed from raw data acquired over an angular range less than an angular range of the resultant image.

Then, the tomographic image processing apparatus 300 generates a third partial reconstruction image by using a first partial reconstruction image corresponding to the first angular range of the previous rotation period and the second partial reconstruction image at operation S406). An X-ray generator and an X-ray detector may rotate along a predetermined trajectory with a specific period. In an embodiment, the rotation period refers to a rotation period with which the X-ray generator and the X-ray detector rotate. The first partial reconstruction image may be an image reconstructed from raw data acquired in the first angular range during a rotation period previous to a rotation period corresponding to the second partial reconstruction image. The first partial reconstruction image may be stored in the memory 330, and the processor 320 may use the stored first partial reconstruction image for generating the third partial reconstruction image. The tomographic image processing apparatus 300 may generate the third partial reconstruction image based on the first and second partial reconstruction images. The first and second partial reconstruction images may be synthesized using an averaging synthesis or weighted averaging synthesis method. The tomographic image processing apparatus 300 may generate the third partial reconstruction image by synthesizing the first and second partial reconstruction images.

Next, the tomographic image processing apparatus 300 may store the third partial reconstruction image in the memory 330 at operation S408. The second partial reconstruction image may be converted into the third partial reconstruction image before being stored in the memory 330, and the third partial reconstruction image may then be stored in the memory 330. According to an embodiment, when the third partial reconstruction image is stored in the memory 330, the first partial reconstruction image is then deleted from the memory 330.

Lastly, the tomographic image processing apparatus 300 generates a resultant image based on a plurality of partial reconstruction images at operation S410). A plurality of partial reconstruction images are stored in the memory 330, and the processor 320 generates a resultant image by synthesizing the partial reconstruction images stored in the memory 330. According to an embodiment, the processor 320 generates a resultant image by summing a preset number of partial reconstruction images.

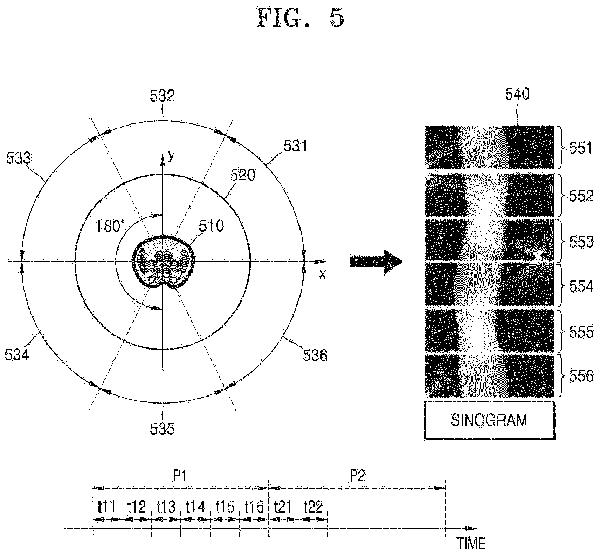

FIG. 5 illustrates a process of acquiring raw data according to an embodiment.

According to an embodiment, the X-ray generator and the X-ray detector may rotate along a specific trajectory 520. The X-ray generator and the X-ray detector may rotate to scan an object 510 only when a user inputs a scan control signal via an input interface. The X-ray generator and the X-ray detector may continuously rotate along the specific trajectory 520 while the scan control signal is being input. FIG. 5 shows an example in which the specific trajectory 520 has an angular range of 360°, however an angular range for the specific trajectory 520 may vary according to an embodiment. For example, a CT system may use a specific trajectory 520 having only an angular range of 180°, or when the CT system is implemented as a C-arm CT system, a specific trajectory 520 may have an angular range that is greater than or equal to 180° but less than 360°. In this case, the X-ray generator and the X-ray detector may be used to scan the object 510 with a specific period while reciprocating within the C-arm structure. In the present specification, an angular range for a trajectory along which the X-ray generator and the X-ray detector move may be referred to as 'the entire angular range'. A resultant image corresponds to the entire angular range, and a rotation period may be a period during which the object 510 is scanned over the entire angular range one time.

The data acquisition interface 310 receives raw data 540 generated by scanning the object 510. For example, the raw data 540 may be in the form of a sinogram as shown in FIG. 5. As a scan of the object 510 proceeds, a sinogram is input for each phase and accumulated.

Partial angular ranges, for example first angular range 531, second angular range 532, third angular range 533, fourth angular range 534, fifth angular range 535, and sixth angular range 536, are defined by partitioning the entire angular range into a predetermined number of smaller angular ranges. The first through sixth angular ranges 531 through 536 may have the same angular interval therebetween and may be defined not to overlap one another. The sum of the first through sixth angular ranges 531 through 536 may form the entire angular range. Furthermore, the first through sixth angular ranges 531 through 536 may be defined by uniformly partitioning the entire angular range. Although the disclosure includes description of an embodiment wherein the entire angular range is 360° and each partial angular range is 60°, the partial angular range may be determined differently according to embodiments.

According to an embodiment, the user may adjust a partial angular range by directly setting the partial angular range or setting a parameter related to the partial angular range. According to another embodiment, the tomographic image processing apparatus 300 may adjust a partial angular range based on the type of a scanning protocol, the type of the object 510, whether the object 510 is rigid or non-rigid, etc. For example, a partial angular range may be set to be smaller with respect to a non-rigid object than with respect to a rigid object.

The object 510 may be scanned with a plurality of rotation periods, and scans may be performed sequentially and iteratively over each of the first through sixth angular ranges 531 through 536. For example, scans may be respectively performed over the first through sixth angular ranges 531 through 536 in eleventh time interval t11, twelfth time interval t12, thirteenth time interval t13, fourteenth time interval t14, fifteenth time interval t15, and sixteenth time interval t16. Subsequently, scans may be respectively performed over the first and second angular ranges 531 and 532 during twenty-first time interval t21 and twenty-second time interval t22.

As scans of the object 510 proceed, the sinogram raw data 540 may include pieces of data respectively corresponding to the first through sixth angular ranges 531 through 536. For example, during a first rotation period P1, first raw data 551, second raw data 552, third raw data 553, fourth raw data 554, fifth raw data 555, and sixth raw data 556 may respectively correspond to the scans performed over the first through sixth angular ranges 531 through 536 in the eleventh through sixteenth time intervals t11 through t16. Subsequently, during a second rotation period P2, seventh and eighth raw data may be continuously acquired by respectively performing the scans over the first and second angular ranges 531 and 532 during the twenty-first and twenty-second time intervals t21 and t22.

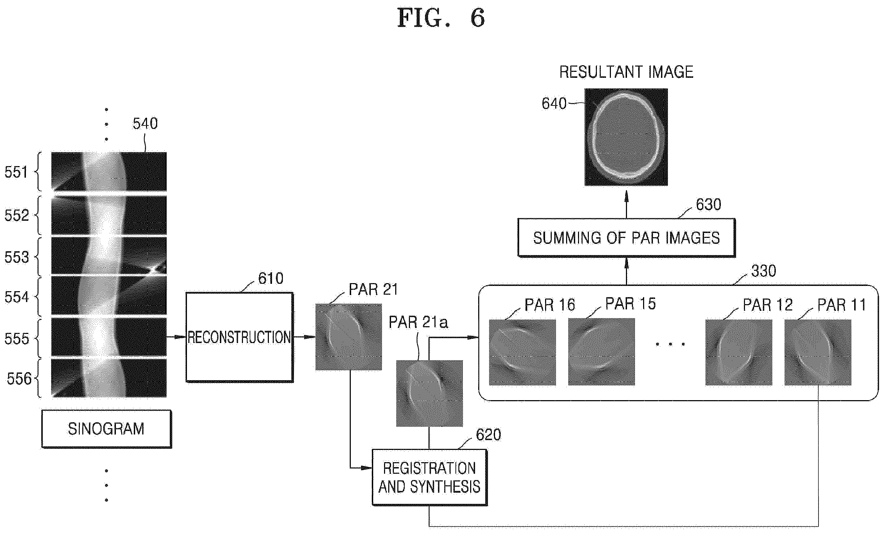

FIG. 6 illustrates a process of generating a resultant image based on first and second partial reconstruction images, according to an embodiment.

When a sinogram raw data 540 is input, the processor 320 performs a reconstruction process 610 for generating a partial reconstruction image each time raw data is input at first angular intervals. The processor 320 may respectively generate eleventh PAR image PAR 11, twelfth PAR image PAR 12, thirteenth PAR image PAR 13, fourteenth PAR image PAR 14, fifteenth PAR image PAR 15, and sixteenth PAR image PAR 16 respectively from first through sixth raw data 551 through 556. Subsequently, the processor 320 may respectively generate twenty-first PAR image PAR 21 and twenty-second PAR image PAR 22 from seventh and eighth raw data. The processor 320 may sequentially store a plurality of PAR images in the memory 330. The processor 320 may control the memory 330 to sequentially store the plurality of PAR images.

According to an embodiment, the memory 330 may be a queue memory operating in a FIFO mode. In an embodiment wherein the first angular interval is 60° and the entire angular range is 360°, the memory 330 may store a total of six partial reconstruction images. Each time a new partial reconstruction image is input to the memory 330, the oldest partial reconstruction image may be discarded from the memory 330. A new partial reconstruction image and a discarded partial reconstruction image are hereinafter referred to as a new PAR image and an existing PAR image, respectively. In this case, the existing PAR image is obtained during a rotation period different from that for the new PAR image but corresponds to the same angular range as the new PAR image. Referring to FIG. 6, the existing PAR image is the eleventh PAR image PAR 11 generated from the first raw data 551 that is acquired by scanning over the first angular range 531 in eleventh time interval t11, and the new PAR image is the twenty-first PAR image PAR 21 generated from the seventh raw data that is acquired by scanning over the first angular range 531 in twenty-first time interval t21.

After generating the new PAR image (twenty-first PAR image PAR 21), the processor 320 performs registration and synthesis 620 between the new PAR image and the existing PAR image (eleventh PAR image PAR 11). The processor 320 may register the existing PAR image (PAR 11) to the new PAR image (PAR 21). The synthesis may include processes such as averaging synthesis, weighted averaging synthesis, etc. A synthesized PAR image PAR 21a generated by performing the registration and synthesis 620 is input to the memory 330 and the existing PAR image (PAR 11) is thereafter deleted from the memory 330.

According to an embodiment, when the existing PAR image obtained during the previous rotation period is not stored in the memory 330, the processor 320 stores the new PAR image in the memory 330 without performing synthesis. A PAR image obtained during the previous rotation period is not stored in the memory 330 until a predetermined time elapses after a scan of an object starts. Thus, when the existing PAR image obtained during the previous rotation period is not stored in the memory 330, the processor 320 stores the new PAR image in the memory 330 without performing the registration and synthesis 620 with the existing PAR image. Otherwise, when the existing PAR image obtained during the previous rotation period is stored in the memory 330, the processor 320 stores in the memory 330 the synthesized PAR image PAR 21a obtained after performing the registration and synthesis 620 between the existing PAR image and the new PAR image. According to an embodiment, the processor 320 may operate so as not to perform registration and synthesis of PAR images until an X-ray generator completes its rotation over one rotation period and to perform the registration and synthesis of PAR images after the X-ray generator completes its rotation over one rotation period.

When the synthesized PAR image PAR 21a is input to the memory 330, the processor 320 performs a process 630 of generating a resultant image 640 by summing six PAR images stored in the memory 330. According to an embodiment, the process 630 of generating the resultant image 640 may be performed each time the synthesized PAR image PAR 21a is input to the memory 330. According to another embodiment, the process 630 of generating the resultant image 640 may be performed every predetermined period. For example, the predetermine period may be set to one rotation period, a plurality of rotation periods, or the like.

According to an embodiment, the processor 320 may include a first processor for performing the registration and synthesis 620 and a second processor for performing the process 630 of generating the resultant image 640 by summing the PAR images. According to an embodiment, the processor 320 may further include a third processor for performing the reconstruction process 610.

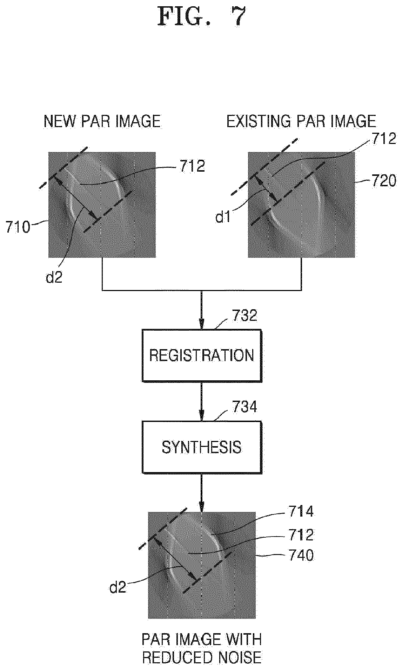

FIG. 7 illustrates a process of performing registration and synthesis, according to an embodiment.

According to an embodiment, when a new PAR image 710 is input, the new PAR image 710 is registered and synthesized with an existing PAR image 720. When embodiments are used in CT fluoroscopy as shown in FIG. 2, a real-time CT image is provided to a user during a procedure or surgery. As various tools are used for a procedure or surgery, the real-time CT image shows the tools used for the procedure or surgery. As a procedure or surgery proceeds, a position of a tool changes. For example, when the user inserts an injection needle 712 into an object, a position of the injection needle 712 changes over time, and this change in position is reflected in the real-time CT image. For example, the existing PAR image 720 shows an injection needle being inserted into the object and pushed to a depth d1, while the new PAR image 710 obtained after one rotation period shows the injection needle being inserted into the object and pushed to a depth d2 that is greater than the depth d1. According to embodiments, because a processing time has to be shortened to provide a real-time CT image showing progress of the procedure or surgery, a resultant image is generated using partial reconstruction images. However, a partial reconstruction image may have a poor quality because the amount of accumulated data therein is smaller than that in an image obtained over the entire angular range. According to embodiments, a SNR in a region other than the injection needle 712 may be improved by synthesizing the existing PAR image 720 into the new PAR image 710, and accordingly, the image quality of the real-time CT image may be improved.

According to an embodiment, the processor 320 first registers the existing PAR image 720 and new PAR image 710 with reference to the new PAR image 710 at operation 732. The processor 320 may register the new PAR image 710 and existing PAR image 720 based on surface information represented in the new PAR image 710 and existing PAR image 720. The surface information may be acquired based on edges of the new PAR image 710 and existing PAR image 720.

According to an embodiment, the processor 320 may perform rigid or non-rigid registration according to the type of the object. When the object is a stationary object such as the liver or brain, the processor 320 may perform rigid registration. When the object is a moving object such as the heart, the processor 320 may perform non-rigid registration. The processor 320 may identify the type of the object based on a scanning protocol. As another example, the processor 320 may identify the type of the object based on a resultant image. As another example, the processor 320 may identify the type of the object according to a user input.

According to an embodiment, the processor 320 may perform registration by downsampling the new PAR image 710 and existing PAR image 720. Image registration may be a process requiring a high processing load. Thus, according to an embodiment, a processing time may be shortened by performing downsampling on an image for registration, thereby reducing the delay time in providing a real-time image.

When the registration is completed, the processor 320 performs image synthesis of the new PAR image 710 and existing PAR image 720 obtained after the registration. The image synthesis may be performed via averaging synthesis or weighted averaging synthesis. When weighted averaging synthesis is performed, weights may be determined according to motion information, the type of the object, and whether the object is rigid or non-rigid. For example, when the degree of motion exceeds a reference value, a weight of the new PAR image 710 may be set higher than that of the existing PAR image 720. A difference in weight may vary depending on the degree of motion.

When the object is a non-rigid, the processor 320 may set a weight of the new PAR image 710 to be higher than a weight of the existing PAR image 720. Furthermore, the processor 320 may adjust a weight based on information indicating a non-rigid motion. For example, when the object is the heart, the processor 320 may adjust a weight based on electrocardiography (ECG) information. For example, when there is a large difference between heartbeat phases respectively corresponding to the new PAR image 710 and the existing PAR image 720, a difference between weights of the new and existing PAR images 710 and 720 may be increased. Otherwise, when there is a small difference between the heartbeat phases, the difference between the weights of the new and existing PAR images 710 and 720 may be reduced.

When the synthesis is completed, a synthesized partial reconstruction image 740 with reduced noise is generated. In the synthesized partial reconstruction image 740, a SNR in a portion of an anatomical structure 714 of a human body may be increased.

Furthermore, because a procedural or surgical instrument has a much higher CT number than the human body, pixel values of the procedural or surgical instrument in the new PAR image 710 may be preserved in a display image even after the image synthesis 734 is performed. A CT image may be expressed as CT numbers, and the number of CT numbers is greater than the number of gray levels provided by a display device. Thus, a display image for a CT image may be generated by mapping some of the CT numbers to the same value. In this case, more gray levels are assigned to a CT number range corresponding to the human body while fewer gray levels are assigned to CT numbers not corresponding to the human body. In this case, a range of CT numbers to which gray levels are to be assigned may be defined by setting a window level and a window width. Because a metal is not a constituent of the human body, a CT number of the metal is usually outside the widow level and the window width. Due to this, a CT number of a metal portion is outside of the window level and the window width before and after the image synthesis 734 of the new PAR image 710 and existing PAR image 720. Therefore, because the CT number of the metal portion is likely to correspond to the same or nearly identical gray level in a display image and thus, pixel values of the metal portion in the display image may be preserved.

In particular, a real-time CT image may be mainly viewed as a guide during insertion of a procedural or surgical instrument. In an interval when the real-time image is mainly used as a guide, insertion of the procedural or surgical instrument in the new PAR image 710 may progress farther than in the existing PAR image 720. Furthermore, position information of the procedural or surgical instrument in the new PAR image 710 is preserved in the synthesized partial reconstruction image 740 as well.

According to an embodiment, the processor 320 may perform synthesis of the new PAR image 710 and existing PAR image 720 during insertion of a procedural or surgical instrument, or may not perform the synthesis during removal of the procedural or surgical instrument. The insertion and removal of an instrument may be determined based on movement of a distal end of the instrument in a PAR image.

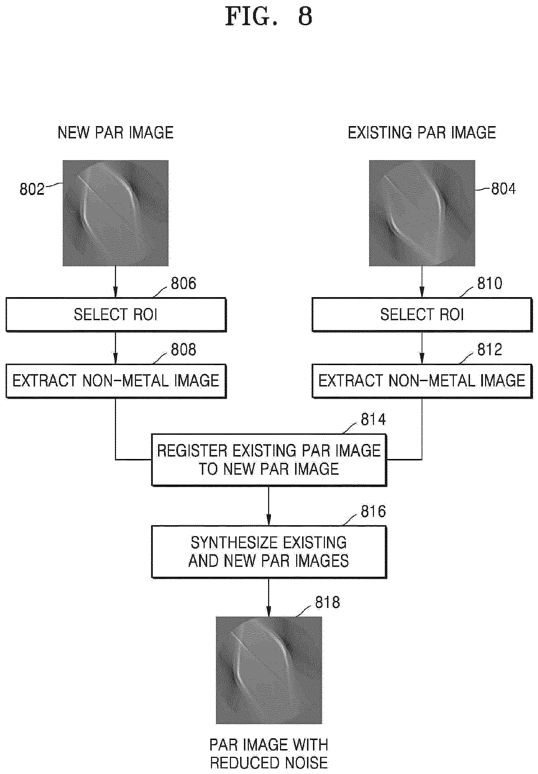

FIG. 8 illustrates a process of synthesizing a new PAR image and an existing PAR image, according to an embodiment.

The processor 320 selects a region of interest (ROI) in the new PAR image 802 in operation 806, and selects an ROI in the existing PAR image 804 in operation 810. For example, an ROI may correspond to a region of a procedural or surgical instrument. The processor 320 may select an ROI based on a user input or a reconstruction image. For example, a user may select, in a resultant image, a region corresponding to a procedural or surgical instrument, and the processor 320 may select an ROI by tracking the region selected by the user. As another example, the tomographic image processing apparatus 300 may provide a user interface (UI) for selecting the type of ROI, for example a metal needle, a non-metal needle, a hose, etc.. The user may select a type of ROI via the UI, and the processor 320 may detect, in a partial reconstruction image, a region corresponding to the type selected by the user and select the region as an ROI. The processor 320 may define and select an ROI based on a CT number, a shape, etc., of an instrument of the type selected by the user.

Then, the processor 320 respectively extracts non-metal images from a new PAR image 802 at operation 808, and from an existing PAR image 804 at operation 812. The non-metal images are captured of a region corresponding to a body part and may be extracted based on a CT number of the body part. As another example, the non-metal images may be extracted by respectively removing the ROIs from the new and existing PAR images 802 and 804. For example, when CT fluoroscopy is used to capture images of a process of performing biopsy with a metal instrument, the non-metal images may be extracted by respectively removing portions corresponding to CT numbers of a metal from the new PAR image 802 and existing PAR image 804.

Then, the processor 320 registers the existing PAR image 804 to the new PAR image 802 at operation 814. In this case, the processor 320 may perform image registration based on the extracted non-metal images.

Then, the processor 320 synthesizes the existing PAR image 804 with the new PAR image 802 at operation 816. In this case, the processor 320 may perform averaging or weighted averaging based on the non-metal images obtained after the registration and generate a synthesized PAR image 818 with a reduced noise by synthesizing the ROI in the new PAR image 802 into an image obtained after the averaging or weighted averaging. The processor 320 may generate a first intermediate image by performing synthesis based on the non-metal image and obtain the synthesized PAR image 818 with a reduced noise by synthesizing the ROI in the new PAR image 802 into the first intermediate image.

According to an embodiment, the processor 320 may extract a non-metal image from the existing PAR image 804 and synthesize the non-metal image from the existing PAR image 804 with the entire region of the new PAR image 802. In this case, the processor 320 may generate the synthesized PAR image 818 with a reduced noise directly from an image obtained by synthesizing the non-metal image from the existing PAR image 804 and the new PAR image 802 without synthesizing the ROI in the new PAR image 802 with the non-metal image obtained after synthesis.

According to the embodiment, by performing registration and synthesis based on an image excluding an ROI, it is possible to improve a SNR in a body structure region while preserving data values of a region of a procedural or surgical instrument corresponding to the ROI. Tomographic image processing apparatuses and methods according to embodiments may be used to allow the user to observe movement of a tool used in a biopsy procedure, etc., within a body. According to the embodiment, a region other than an ROI corresponding to an instrument used for biopsy is extracted and synthesized and then the ROI is synthesized into an image obtained after the synthesis, thereby allowing accurate visualization of the ROI without loss of data of the ROI due to the synthesis .

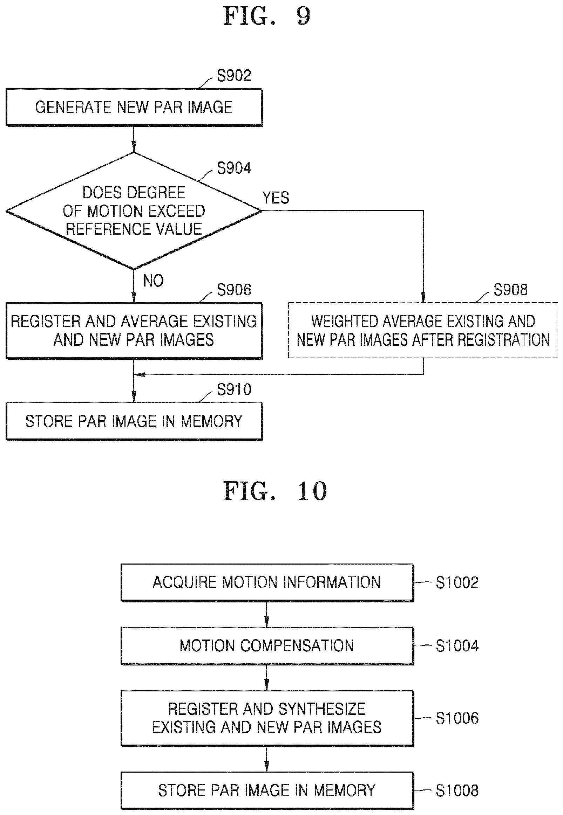

FIG. 9 is a flowchart of a process of registering and synthesizing partial reconstruction images, according to an embodiment.

The processor 320 generates a new PAR image at operation S902 and calculates a motion value representing the degree of motion of an object from the new PAR image and an existing PAR image. The motion value may be a value representing the motion of a surface of the object.

The processor 320 may calculate the motion value based on an edge of an image. The processor 320 may acquire motion information by registering the new and existing PAR images. For example, the processor 320 may register the existing PAR image and the new PAR image with reference to the new PAR image and calculate a motion vector representing a direction and a magnitude of motion of each pixel in the existing PAR image. The motion vector corresponds to motion information.

According to another embodiment, the object is the heart, and a motion value may be calculated based on an ECG signal. According to another embodiment, the motion value may be calculated based on a motion sensor attached to the object.

The processor 320 determines whether a motion value exceeds a reference value at operation S904. The reference value may be a predetermined value. The reference value may be determined differently according to the type of the object or which body part corresponds to the object.

When the motion value does not exceed the reference value in operation S904, the processor 320 performs registration and averaging synthesis of the existing PAR image and the new PAR image at operation S906. The processor stores a partial reconstruction image obtained after the averaging synthesis in the memory 330 at operation S910

Otherwise, when the motion value exceeds the reference value in operation S904, according to an embodiment, the processor 320 stores the new PAR image in the memory 330 without synthesizing the new and existing PAR images at operation S910.

According to another embodiment, when the motion value exceeds the reference value in operation S904, the processor 320 registers the existing and new PAR images and then synthesizes them via weighted averaging at operation S908. A partial reconstruction image obtained after the synthesis via weighted averaging is stored in the memory 330 at operation S910. According to the embodiment, a weight of the new PAR image is set higher than a weight of the existing PAR image. According to an embodiment, the larger the motion value is, the higher a weight of the new PAR image may be set.

According to another embodiment, the reference value may include a first reference value and a second reference value greater than the first reference value. When the motion value is greater than or equal to the second reference value, the processor 320 may store the new PAR image in the memory 330 without synthesizing the new PAR image and existing PAR image. When the motion value is greater than or equal to the first reference value but less than the second reference value, the processor 320 may perform weighted averaging synthesis by setting a weight of the new PAR image to be higher than a weight of the existing PAR image.

FIG. 10 is a flowchart of a method of registering and synthesizing partial reconstruction images, according to an embodiment.

According to an embodiment, when the new PAR image is generated, motion information indicating motion between the existing PAR image and new PAR image may be acquired at operation S1002 and motion compensation may be performed on the existing PAR image at operation S1004. The motion compensation may be performed such that a body surface in the existing PAR image is moved with respect to the new PAR image. The motion information may be expressed in motion vectors. The processor 320 may move a surface of a human body in the existing PAR image to correspond to motion vectors. According to an embodiment, the processor 320 may respectively extract ROIs from the new and existing PAR images and acquire motion information with respect to regions other than the ROIs.

When the motion compensation is performed on the existing PAR image in operation S1004, the processor 320 performs registration and synthesis with the new PAR image by using the existing PAR image that has undergone the motion compensation at operation S1006. Furthermore, the processor 320 stores a partial reconstruction image obtained after the synthesis in the memory 330 (S1008).

According to the embodiment, when the object moves, a SNR in a body structure region may be improved by performing motion compensation.

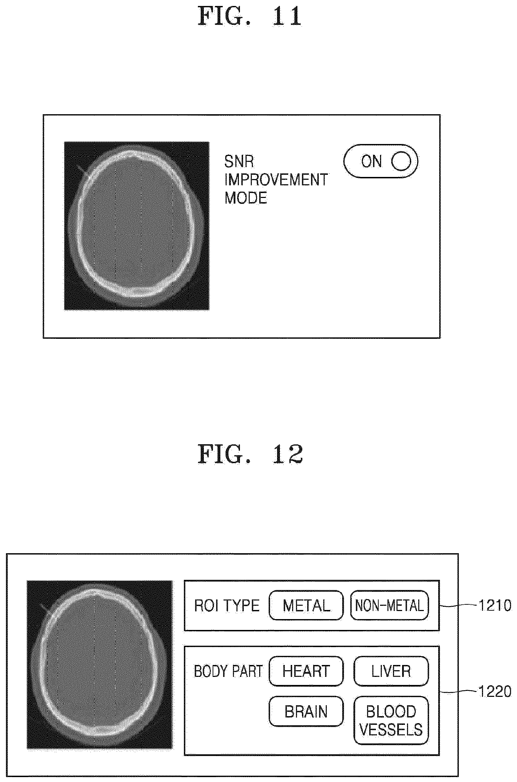

FIG. 11 illustrates a UI view according to an embodiment.

According to an embodiment, the tomographic image processing apparatus 300 may perform synthesis of a previous PAR image and a new PAR image only when a user selects a predetermined mode. For example, the user may select a SNR improvement mode via a graphical UI (GUI), and the tomographic image processing apparatus 300 may perform synthesis of the previous and new PAR images only when the user selects the SNR improvement mode. Otherwise, when the user does not select the SNR improvement mode, the processor 320 stores the new PAR image in the memory 330 without synthesis of the previous and new PAR images.

According to the embodiment, the tomographic image processing apparatus 300 may further include a display for providing a GUI view and an input device for receiving a user input. For example, the input device may be implemented in the form of a key, a button, a touch screen, a touch pad, etc.

FIG. 12 illustrates a UI view according to an embodiment.

According to an embodiment, the tomographic image processing apparatus 300 may provide a first GUI 1210 for selecting an ROI type. For example, the user may select whether an ROI is a metal or non-metal via the first GUI 1210. The processor 320 may extract the ROI by determining a CT number range of the ROI based on the ROI type selected by the user.

According to an embodiment, the tomographic image processing apparatus 300 may provide a second GUI 1220 for selecting a part of an object. For example, the user may select, via the second GUI 1220, which of body parts such as the heart, the liver, the brain, and blood vessels corresponds to a part of the object. The processor 320 may obtain information about a shape of the object based on the part of the object selected by the user and perform registration with respect to the object based on the information about the shape of the object. Furthermore, the processor 320 may perform rigid or non-rigid registration and motion compensation based on information about the part of the object.

Either or both of the first GUI 1210 and second GUI 1220 may be provided.

According to the embodiment, the tomographic image processing apparatus 300 may further include a display for providing a GUI view and an input device for receiving a user input. For example, the input device may be implemented in the form of a key, a button, a touch screen, a touch pad, etc.

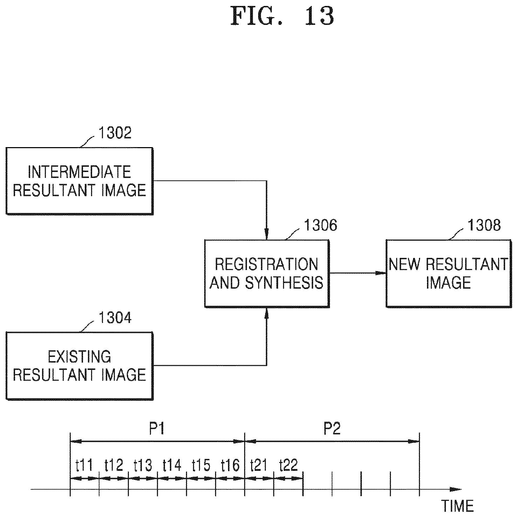

FIG. 13 illustrates a process of synthesizing an intermediate resultant image 1302 and an existing resultant image 1304, according to an embodiment.

According to an embodiment, when the intermediate resultant image 1302 is generated by summing PAR images, the processor 320 generates a new resultant image 1308 by performing registration and synthesis 1306 of the intermediate resultant image 1302 and existing resultant image 1304. In this case, the existing resultant image 1304 is a resultant image generated during a previous rotation period P1. The intermediate resultant image 1302 and new resultant image 1308 may be resultant images corresponding to a current rotation period P2, and the existing resultant image 1304 may be a resultant image corresponding to the previous rotation period P1. In the description of the embodiment, referring to FIG. 6, the resultant image 640 generated by summing the PAR images at operation 630 obtained over the current rotation period P2 is referred to as the intermediate resultant image 1302, and a resultant image previously generated during the previous rotation period P1 is referred to as the existing resultant image 1304.

According to an embodiment, the existing resultant image 1304 may be a resultant image generated before one or more rotation periods.

According to an embodiment, the new resultant image 1308 may be generated each time a partial image is generated, each time a plurality of partial images are generated, or every rotation period. The registration and synthesis of the intermediate resultant image 1302 and existing resultant image 1304 may be performed each time the new resultant image 1308 is generated.

Embodiments with respect to the above-described storage, registration, and synthesis of PAR images may be applied to the process of generating the new resultant image 1308 from the intermediate resultant image 1302 and existing resultant image 1304. For example, the existing resultant image 1304 may be stored in a queue memory, and the registration and synthesis 1306 of the intermediate resultant image 1302 and existing resultant image 1304 may be performed before the existing resultant image 1304 is deleted from the queue memory. As the new resultant image 1308 generated by performing the registration and synthesis 1306 is stored in the queue memory, the existing resultant image 1304 may be deleted therefrom. Furthermore, as described with reference to FIG. 7, the registration 732 and the image synthesis 734 may be sequentially performed on the intermediate resultant image 1302 and existing resultant image 1304. Furthermore, as described with reference to FIG. 8, the processor 320 may respectively extract non-metal images from the intermediate resultant image 1302 and existing resultant image 1304 to perform registration and synthesis of the intermediate resultant image 1302 and existing resultant image 1304 based on the extracted non-metal images and then synthesize an ROI in the intermediate resultant image 1302 to generate the new resultant image 1308. Furthermore, as described with reference to FIG. 9, when the degree of motion between the existing and intermediate resultant images 1304 and 1302 exceeds a reference value, the processor 320 may generate the new resultant image 1308 without synthesizing the existing and intermediate resultant images 1304 and 1302 or by weighted averaging them. Furthermore, the processor 320 may acquire information about motion of the intermediate resultant image 1302 with respect to the existing resultant image 1304 and perform registration and synthesis of the intermediate resultant image 1302 and existing resultant image 1304 after performing motion compensation on the existing resultant image 1304.

According to an embodiment, the processor 302 may perform registration and synthesis of partial images as well as registration and synthesis of resultant images. According to another embodiment, the processor 302 may perform registration and synthesis of only partial images and not for resultant images. According to another embodiment, the processor 302 may perform only registration and synthesis of resultant images and not for partial images.

According to the embodiment, a SNR of resultant images may be improved by performing registration and synthesis of the resultant images.

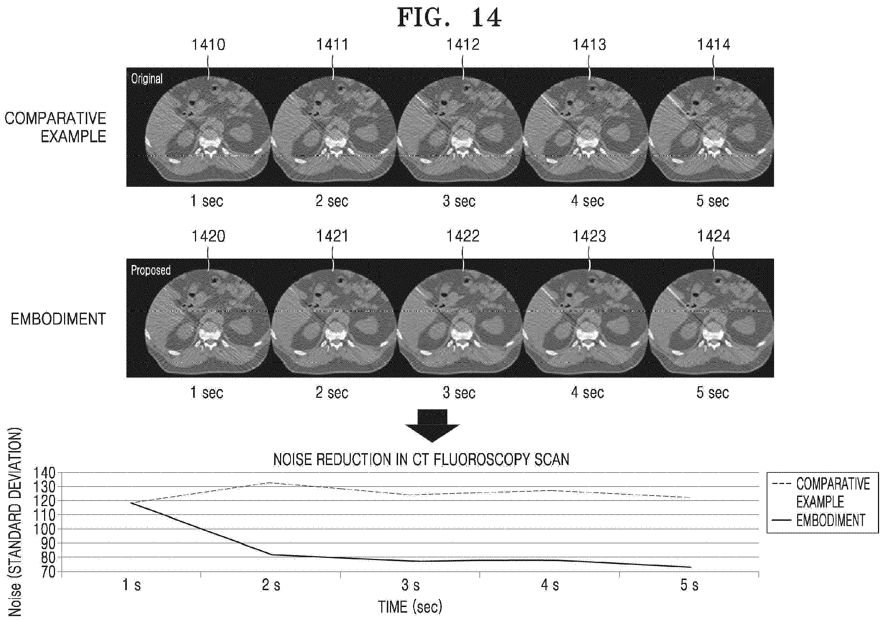

FIG. 14 illustrates an effect of a method according to an embodiment compared to a method of the related art when using simulation data acquired without motion.

According to embodiments, referring to FIG. 7, as the existing PAR image 720 is synthesized into the new PAR image 710, noise in a region other than the injection needle 712 is reduced, and simulation data was created to verify such noise reduction. In order to create the simulation data, virtual patient data that may be acquired by taking X-rays for a total of 5 seconds was first generated by copying actual patient data acquired by taking X-rays for 1 second without motion. Furthermore, data was generated by simulating the movement of inserting a needle-shaped structure having a Hounsfield unit (HU) value similar to that of an actual structure over time and mathematically X-raying the movement and then added to the virtual patient data. The simulation data created via the above process was used to compare per second the extents of noise reduction according to a comparative example and according to an embodiment. Referring to FIG. 14, resultant image 1410, resultant image 1411, resultant image 1412, resultant image 1413, and resultant image 1414 reconstructed from the simulation data according to the comparative example are sequentially shown at one-second intervals, and resultant image 1420, resultant image 1421, resultant image 1422, resultant image 1423, and resultant image 1424 reconstructed from the simulation data according to the embodiment are sequentially shown at one-second intervals.

A graph of FIG. 14 illustrates noise values respectively detected in the resultant images 1410 through 1414 according to the comparative example and the resultant images 1420 through 1424 according to the embodiment. In the graph of FIG. 14, the abscissa and ordinate respectively denote time and a standard deviation of noise values. Referring to the graph of FIG. 14, the extent of noise reduction according to the comparative example remained almost constant over time while the extent of noise reduction according to the embodiment increased continuously over time.

The embodiments may be implemented as a software program including instructions stored in a computer-readable storage medium.

A computer may refer to a device configured to retrieve an instruction stored in the computer-readable storage medium and to operate, in response to the retrieved instruction, and may include an tomographic imaging apparatus according to embodiments.

The computer-readable storage medium may be provided in the form of a non-transitory storage medium. In this regard, the term 'non-transitory' means that the storage medium does not include a signal and is tangible, and the term does not distinguish between data that is semi-permanently stored and data that is temporarily stored in the storage medium.

In addition, the tomographic imaging apparatus or the method of controlling the tomographic imaging apparatus according to embodiments may be provided in the form of a computer program product. The computer program product may be traded, as a product, between a seller and a buyer.

The computer program product may include a software program and a computer-readable storage medium having stored thereon the software program. For example, the computer program product may include a product, for example a downloadable application, in the form of a software program electronically distributed by a manufacturer of the tomographic imaging apparatus or through an electronic market, for example Google ™, Play Store ™, and App Store ™. For such electronic distribution, at least a part of the software program may be stored on the storage medium or may be temporarily generated. In this case, the storage medium may be a storage medium of a server of the manufacturer, a server of the electronic market, or a relay server for temporarily storing the software program.

In a system including a server and a terminal, for example the tomographic imaging apparatus, the computer program product may include a storage medium of the server or a storage medium of the terminal. Alternatively, in a case where a third device, for exmaple a smartphone, that communicates with the server or the terminal is present, the computer program product may include a storage medium of the third device. Alternatively, the computer program product may include a software program that is transmitted from the server to the terminal or the third device or that is transmitted from the third device to the terminal.

In certain embodiments according to this disclosure, one of the server, the terminal, and the third device may execute the computer program product, thereby performing the method according to embodiments. Alternatively, at least two of the server, the terminal, and the third device may execute the computer program product, thereby performing the method according to embodiments in a distributed manner.

For example, the server, for example a cloud server, an artificial intelligence (AI) server, or the like, may execute the computer program product stored in the server, and may control the terminal to perform the method according to embodiments, the terminal communicating with the server.

As another example, the third device may execute the computer program product, and may control the terminal to perform the method according to embodiments, the terminal communicating with the third device. In more detail, the third device may remotely control the tomographic imaging apparatus to emit X-ray to an object, and to generate an image of an inner part of the object, based on detected radiation which passes the object and is detected in an X-ray detector.

As another example, the third device may execute the computer program product, and may directly perform the method according to embodiments, based on at least one value input from an auxiliary device, for example a gantry of CT system. In more detail, the auxiliary device may emit X-ray to an object and may obtain information of radiation which passes the object and is detected in an X-ray detector. The third device may receive an input of signal information about the detected radiation from the auxiliary device, and may generate an image of an inner part of the object, based on the input radiation information.

In a case where the third device executes the computer program product, the third device may download the computer program product from the server, and may execute the downloaded computer program product. Alternatively, the third device may execute the computer program product that is pre-loaded therein, and may perform the method according to the embodiments.

According to embodiments, an image with reduced noise may be provided by reducing noise in a tomographic image generated using a partial reconstruction method.

While embodiments of the present disclosure have been particularly shown and described with reference to the accompanying drawings, it will be understood by those of ordinary skill in the art that various changes in form and details may be made therein without departing from the spirit and scope as defined by the appended claims. The disclosed embodiments should be considered in descriptive sense only and not for purposes of limitation.

## Claims

1. A tomographic image processing apparatus comprising:
a data acquisition interface configured to acquire raw data;
a memory; and
at least one processor configured to:
obtain, from the memory, a first partial reconstruction image corresponding to a partial angular range of a first rotation period of an X-ray generator;
generate a second partial reconstruction image from partial raw data acquired in a partial angular range of a second rotation period of the X-ray generator, wherein the partial angular range of the first rotation period corresponds to the partial angular range of the second rotation period;
generate a third partial reconstruction image based on the first partial reconstruction image and the second partial reconstruction image;
store the third partial reconstruction image in the memory; and
generate a resultant image based on the third partial reconstruction image and a plurality of partial reconstruction images stored in the memory.

2. The tomographic image processing apparatus of claim 1,
wherein the plurality of partial reconstruction images respectively correspond to a plurality of angular ranges with a same angular interval therebetween,
wherein a sum of the plurality of angular ranges corresponding to the plurality of partial reconstruction images corresponds to an angular range of the resultant image, and
wherein the at least one processor is further configured to generate the resultant image by summing the plurality of partial reconstruction images.

3. The tomographic image processing apparatus of claim 1,
wherein the memory comprises a queue memory operating in a first-in-first-out (FIFO) mode, and includes a storage space corresponding to a capacity for storing a predetermined number of the plurality of partial reconstruction images, and
wherein the predetermined number is a number of the partial reconstruction images used to generate the resultant image.

4. The tomographic image processing apparatus of claim 3, wherein the memory is configured to delete the first partial reconstruction image based on the third partial reconstruction image being input.

5. The tomographic image processing apparatus of claim 1, wherein the at least one processor is further configured to:
register the first partial reconstruction image to the second partial reconstruction image; and
generate the third partial reconstruction image by using the second partial reconstruction image and the first partial reconstruction image after the registering .

6. The tomographic image processing apparatus of claim 1, wherein the at least one processor is further configured to generate the third partial reconstruction image by performing averaging synthesis on the first partial reconstruction image and the second partial reconstruction image.

7. The tomographic image processing apparatus of claim 1, wherein the resultant image includes a metal object.

8. The tomographic image processing apparatus of claim 7, wherein the at least one processor is further configured to:
extract a first non-metal region from the first partial reconstruction image and a second non-metal region from the second partial reconstruction image;
synthesize the first non-metal region with the second non-metal region; and
generate the third partial reconstruction image by using an image obtained by the synthesizing and the second partial reconstruction image.

9. The tomographic image processing apparatus of claim 1, wherein the at least one processor is further configured to:
detect motion information in the first partial reconstruction image and the second partial reconstruction image; and
based on a motion value being greater than or equal to a predetermined reference value, store the second partial reconstruction image in the memory.

10. The tomographic image processing apparatus of claim 1, wherein the at least one processor is further configured to:
acquire motion information indicating motion between the first partial reconstruction image and the second partial reconstruction image; and
based on a motion value of the motion being greater than or equal to a preset reference value, generate the third partial reconstruction image by weighted averaging the first partial reconstruction image and the second partial reconstruction image, wherein a weight of the first partial reconstruction image is lower than a weight of the second partial reconstruction image.

11. The tomographic image processing apparatus of claim 1, wherein the raw data comprises image data regarding a moving body part, and
wherein the at least one processor is further configured to:
compensate for motion of the moving body part in the first partial reconstruction image, based on motion information of the moving body part; and
generate the third partial reconstruction image based on the compensated first partial reconstruction image and the second partial reconstruction image.

12. The tomographic image processing apparatus of claim 1, wherein the at least one processor is further configured to perform a computed tomography (CT) fluoroscopy scan.

13. The tomographic image processing apparatus of claim 1, wherein the partial angular range of the first rotation period is less than an angular range of the resultant image.

14. A tomographic image processing method comprising:
acquiring raw data;
obtaining, from a memory, a first partial reconstruction image corresponding to a partial angular range of a first rotation period of an X-ray generator;
generating a second partial reconstruction image from partial raw data acquired in a partial angular range of a second rotation period of the X-ray generator, wherein the partial angular range of the first rotation period corresponds to the partial angular range of the second rotation period;
generating a third partial reconstruction image based on the first partial reconstruction image and the second partial reconstruction image;
storing the third partial reconstruction image in the memory; and
generating a resultant image based on the third partial reconstruction image and a plurality of partial reconstruction images stored in the memory.

15. A computer program comprising program instructions,
wherein the program instructions, when executed by a processor, cause the processor to perform a tomographic image processing method comprising:
acquiring raw data;
obtaining, from a memory, a first partial reconstruction image corresponding to a partial angular range of a first rotation period of an X-ray generator;
generating a second partial reconstruction image from partial raw data acquired in a partial angular range of a second rotation period of the X-ray generator, wherein the partial angular range of the first rotation period corresponds to the partial angular range of the second rotation period;
generating a third partial reconstruction image based on the first partial reconstruction image and the second partial reconstruction image;
storing the third partial reconstruction image in the memory; and
generating a resultant image based on the third partial reconstruction image and a plurality of partial reconstruction images stored in the memory.
